# EUROPÄISCHE PATENTSCHRIFT

(11) **EP 3 120 712 B1**
(45) Veröffentlichungstag und Bekanntmachung des Hinweises auf die Patenterteilung: **13.09.2017**
(21) Anmeldenummer: 15177806.5
(22) Anmeldetag: 22.07.2015
(51) Int. Cl.: A23L 27/10, B01D 11/02, A61K 36/14, A61K 36/185, A61K 36/73, A61K 36/738

(54) **VERFAHREN ZUR VERBESSERTEN EXTRAKTION VON WACHOLDERBEEREN, HAGEBUTTEN, SANDDORNBEEREN, MEHLBEEREN**
METHOD FOR IMPROVED EXTRACTION OF JUNIPER BERRIES, ROSE HIPS, SEA BUCKTHORN BERRIES, SORBUS
PROCEDE D'EXTRACTION AMELIOREE DE BAIES DE GENIEVRE, DE CYNORHODONS, DE BAIES D'ARGOUSIER ET D'ALISIER

(43) Veröffentlichungstag der Anmeldung: 25.01.2017
(73) Patentinhaber: Evonik Degussa GmbH, 45128 Essen (DE)
(72) Erfinder: WIESMÜLLER, Johann, 84518 Garching (DE); MICHLBAUER, Franz, 84558 Kirchweidach (DE); BAND, Rainer, 81737 München (DE)

(56) Entgegenhaltungen:
- EP-A2- 0 154 258
- WO-A1-2004/026802
- WO-A2-2009/125071
- CN-A- 103 494 848
- XU X ET AL: "Optimization of supercritical carbon dioxide extraction of sea buckthorn (Hippophae thamnoides L.) oil using response surface methodology", LWT- FOOD SCIENCE AND TECHNOLOGY, ACADEMIC PRESS, UNITED KINGDOM, Bd. 41, Nr. 7, 1. September 2008 (2008-09-01), Seiten 1223-1231, XP022650041, ISSN: 0023-6438, DOI: 10.1016/J.LWT.2007.08.002 [gefunden am 2008-05-08]
- DAMJANOVIC B ET AL: "Isolation of essential oil and supercritical carbon dioxide extract of Juniperus communis L. fruits from Montenegro", FLAVOUR AND FRAGRANCE JOURNAL NOVEMBER/DECEMBER 2006 JOHN WILEY AND SONS LTD GB, Bd. 21, Nr. 6, November 2006 (2006-11), Seiten 875-880, XP055225805, DOI: 10.1002/FFJ.1711
- MACHMUDAH ET AL: "Supercritical CO2 extraction of rosehip seed oil: Fatty acids composition and process optimization", JOURNAL OF SUPERCRITICAL FLUIDS, PRA PRESS, US, Bd. 41, Nr. 3, 3. Mai 2007 (2007-05-03), Seiten 421-428, XP022054911, ISSN: 0896-8446, DOI: 10.1016/J.SUPFLU.2006.12.011
- REVERCHON E ED - BOLAÑOS GUSTAVO: "Supercritical fluid extraction and fractionation of essential oils and related products", JOURNAL OF SUPERCRITICAL FLUIDS, PRA PRESS, US, Bd. 10, Nr. 1, 14. April 1997 (1997-04-14) , Seiten 1-37, XP004264796, ISSN: 0896-8446, DOI: 10.1016/S0896-8446(97)00014-4

## Beschreibung

Die vorliegende Erfindung betrifft ein Verfahren zur Gewinnung eines hochreinen Extraktes aus Wacholderbeeren, Sanddornbeeren, Hagebutten oder Mehlbeeren, bevorzugt Wacholderbeeren, durch Extraktion mit überkritischem CO₂.

### Hintergrund der Erfindung

Im Stand der Technik sind eine Vielzahl von Extraktionen von verschiedenen Naturstoffen, wie zum Beispiel Gewürzen oder Beeren, beschrieben. Dabei wird unterkritisches (WO 2004/026802 A1) oder überkritisches (siehe die nachfolgend genannten Dokumente) CO₂ als Extraktionsmittel eingesetzt, wobei beim überkritischen CO₂ zur Erreichung einer optimalen Extraktion der Zusatz eines Kosolvens empfohlen wird.

CN103494848 beschreibt die Extraktion von Sanddornbeeren mittels überkritischem CO₂ und Ethanol als Kosolvenz, gefolgt von säulenchromatografischer Aufreinigung des Extraktes. So beschreibt die WO 2009/051606 A1 ein Verfahren zur Extraktion von Biopolymeren aus der Guayule (*Parthenium argentatum*) und anderen Kautschukpflanzen aus der Familie der *Asteraceae, Euphorbiceae, Campanulaceae, Labiatae* und *Moraceae.* Als Extraktionsmittel wird dabei eine Kombination aus Hexan und überkritischem CO₂ genutzt.

CN102827128, CN 103408524, CN 102827129 und CN 103450135 beschreiben die Extraktion von Flavonolen (Betuletol bzw. Broussoflavonol A) und anderen Naturstoffen aus verschiedenen Pflanzenarten, darunter Arnika, oder auch der Rinde des Papiermaulbeerbaumes (*Broussonetia* papyrifera). Die Extraktion selbst erfolgt mit CO₂, gefolgt von der säulenchromatographischen Konzentration des Extraktes.

PL189884 B1 beschreibt die Extraktion von Walnusskernen mit Kohlenstoffdioxid und Alkohol als Kosolvens.

Die Zugabe von Hexan oder Ethanol als Kosolvens zu CO₂ bei der Extraktion von Zimt beschreibt die KR 20130090988 A.

Der Zusatz eines Kosolvens ist laut Stand der Technik (EP 0 154 258 B1) auch bei der Extraktion von Beeren wie jenen des Wacholders mit überkritischem CO₂ nötig, um akzeptable Extraktionsergebnisse zu erzielen.

EP 0 154 258 B1 beschreibt die Extraktion von Wacholder und Gewürzen mit überkritischen Gasen wie CO₂, N₂O, Butan, Propan, Ethan oder Ethylen. Diese Druckschrift diskutiert, dass gerade bei diesem Extraktionsgut das Problem der gleichzeitigen unerwünschten Extraktion von Stoffen auftritt, die sich im Extrakt wiederfinden. Die Entfernung dieser unerwünschten Stoffe im Extrakt ("Entschleimung") erreicht die EP 0 154 258 B1 dadurch, dass dem Extraktionsmittel CO₂ ein Kosolvens als Schleppmittel (zum Beispiel Hexan oder Ethanol) zugegeben wird.

Die Entschleimung erfolgt im Verfahren nach EP 0 154 258 B1 jedoch zum Preis des Einsatzes einer hohen Menge organischen Kosolvens. Dieses ist einerseits ein ökonomisches Problem, da dadurch ein hoher Verbrauch an Kosolvens nötig ist. Andererseits handelt es sich bei dem Kosolvens sehr häufig um das giftige Hexan. Dieses findet sich dann im Extraktionsrückstand, weshalb dieser als Sondermüll entsorgt werden muss. Eine Aufgabe der vorliegenden Erfindung war es deshalb, ein Verfahren zur Extraktion von zum Beispiel Wacholderbeeren zur Verfügung zu stellen, welches den Verbrauch an organischem Kosolvens verringert und in ökologischer Hinsicht eine Verbesserung darstellt. Dabei sollte die Extraktionseffizienz gleich oder sogar besser sein als jene im Stand der Technik beschriebene.

Zusätzlich dazu ergeben sich im Verfahren gemäß EP 0 154 258 B1 noch weitere Probleme. Bei der Herstellung von Extrakten enthaltend Aromastoffen ist es erwünscht, einen Extrakt mit einem möglichst geringen Zuckergehalt zu erhalten. Bei der Extraktion von Wacholderbeeren stellt dies aber ein Problem dar, wenn mit den Verfahren des Standes der Technik gearbeitet wird. So weisen Extrakte aus Wacholderbeeren, die etwa mit dem in der EP 0 154 258 B1 beschriebenen Verfahren gewonnen werden, hohe Zuckergehalte auf. Für vielerlei Anwendungen ist jedoch ein Extrakt aus Wacholderbeeren mit geringem Zuckergehalt notwendig. Zucker ist für viele Anwendung nachteilig, da er zum Beispiel zu Trübungen in Lösungen führt. Daher ist gerade bei der Aromatisierung alkoholischer Getränke wie Gin ein zuckerfreier Extrakt notwendig.

Daneben wurde festgestellt, dass auch der in der EP 0 154 258 B1 erhaltene Wacholderextrakt selbst mit organischem Kosolvens, zum Beispiel Hexan, belastet ist, welches durch energieaufwändige Vakuumdestillation entfernt werden muss, um auf für die Weiterverarbeitung in Lebensmitteln unbedenkliche Werte zu kommen (< 1000 ppm). Diese Vakuumdestillation bedarf eines zusätzlichen Verfahrensschrittes, was das gesamte Verfahren aufwändiger macht.

Die oben beschriebenen Probleme treten auch bei der Extraktion von den Wacholderbeeren ähnlichen Beeren wie Hagebutten, Sanddornbeeren, Mehlbeeren auf. So weisen auch Extrakte aus diesen Beeren einen hohen Anteil an Schleimstoffen auf, was eine Entschleimung nötig macht. Daneben ist ihr Zuckergehalt mit dem der Wacholderbeeren vergleichbar, womit auch bei diesen Beeren das Bedürfnis, einen möglichst zuckerfreien Extrakt zu gewinnen, besteht.

Die weitere Aufgabe der vorliegenden Erfindung bestand deshalb auch darin, ein Extraktionsverfahren zur Verfügung zu stellen, mit dem aus diesen Beeren, insbesondere Wacholderbeeren, möglichst effizient, das heißt insbesondere unter Einsatz von besonders wenig Extraktionsmittel und Energie, ein zuckerfreier Extrakt mit geringem Restgehalt an organischem Lösungsmittel gewonnen werden kann.

Das erfindungsgemäße Verfahren löst die vorstellig beschriebene Aufgabe überraschend.

### Detaillierte Beschreibung der Erfindung

Die vorliegende Erfindung betrifft demnach ein
1. Verfahren zur Gewinnung eines Extraktes **E_{B}** umfassend Aromastoffe von Beeren **B** ausgewählt aus Wacholderbeeren, Sanddornbeeren, Hagebutten, Mehlbeeren, wobei man
   a. die zerkleinerten oder unzerkleinerten Beeren **B** mit überkritischem CO₂ ohne Zusatz eines Kosolvens extrahiert, wodurch ein fester Extraktionsrückstand **B_{Ex}** sowie ein erster Extrakt umfassend überkritisches CO₂ und Aromastoffe erhalten werden;
   b. mindestens teilweise CO₂ aus dem in Schritt a. erhaltenen ersten Extrakt entfernt, wodurch ein zweiter Extrakt, welcher die Aromastoffe und eine gegenüber dem ersten Extrakt verringerte Menge an CO₂ umfasst, erhalten wird;
   c. den in Schritt b. erhaltenen zweiten Extrakt mit mindestens einem Extraktionsmittel ausgewählt aus der Gruppe bestehend aus Alkan, chloriertem Alkan, Alkohol, Alkancarbonsäureester, aliphatisches Keton, aliphatischer Ether extrahiert, wodurch der Extrakt **E_{B}** erhalten wird.
2. Verfahren nach Punkt 1, wobei die Beeren **B** Wacholderbeeren sind.
3. Verfahren nach einem der Punkte 1 oder 2, wobei man in Schritt b. das CO₂ dadurch zumindest teilweise aus dem ersten Extrakt entfernt, dass der erste Extrakt einer Temperatur von > 0°C bis < 100 °C und einem Druck von 0.1 bar bis 72.9 bar ausgesetzt wird.
4. Verfahren nach einem der Punkte 1 bis 3, wobei das Extraktionsmittel in Schritt c. ausgewählt ist aus der Gruppe bestehend aus Hexan, Ethanol, Propanol, Methylacetat.
5. Verfahren nach Punkt 4, wobei das Extraktionsmittel in Schritt c. Hexan ist.
6. Verfahren nach Punkt 4, wobei das Extraktionsmittel in Schritt c. Ethanol ist.
7. Verfahren nach einem der Punkte 1 bis 6, wobei der Schritt a. bei einer Temperatur im Bereich 31 °C bis < 100 °C und einen Druck im Bereich von 74 bar bis 400 bar durchgeführt wird.
8. Verfahren nach einem der Punkte 1 bis 7, wobei die Beeren **B** in Schritt a. zerkleinert eingesetzt werden.
9. Verfahren nach einem der Punkte 1 bis 8, welches kontinuierlich durchgeführt wird.

Die Erfindung bietet gegenüber dem in der EP 0 154 258 B1 anhand von Wacholder beschriebenen Extraktionsverfahren vielerlei Vorteile: Es ist damit möglich, einen Beerenextrakt zu erhalten, der einen deutlich geringeren Gehalt an Zucker aufweist. Zusätzlich erlaubt das Verfahren auch die effektivere Entfernung von Schleimstoffen unter geringerem Verbrauch von organischen und toxischen Lösungsmitteln wie zum Beispiel Hexan oder Ethanol. Schließlich ist durch das erfindungsgemäße Verfahren ein Extraktionsrückstand **B_{Ex}** erhältlich, der nicht mit Kosolvens belastet ist und somit unbedenklich der Kompostierung zugeführt werden kann. Auch wird durch das erfindungsgemäß Verfahren ein Wacholderextrakt erhalten, der auch ohne einen zusätzlichen Vakuumsdestillationsschritt deutlich weniger mit organischem Lösungsmittel, wie zum Beispiel Hexan, belastet ist.

Im erfindungsgemäßen Verfahren werden Beeren **B** ausgewählt aus der Gruppe bestehend aus Hagebutten, Wacholder, Sanddorn, Mehlbeeren eingesetzt. Dabei wird darauf verwiesen, dass es sich bei diesen nicht unbedingt um "Beeren" im botanischen Sinne handeln muss - der Einfachheit halber wird im Rahmen der Erfindung allerdings für Hagebutten, Wacholderbeeren, Sanddornbeeren, Mehlbeeren der Sammelbegriff "Beeren **B**" verwendet.

Als Hagebutten sind erfindungsgemäß die Sammelnussfrüchte der Rosenarten (*Rosa*) zu verstehen. Insbesondere bezeichnet der Begriff "Hagebutte" im Sinne der Erfindung die Sammelnussfrüchte der folgenden Rosen: Hundsrose (*Rosa canina*; *Rosa canina* ssp *Lito*), Gebirgsrose (*Rosa pendulina*), Kartoffelrose (*Rosa rugosa*), Schottische Zaunrose (Rosa *rubiginosa*),

Als Mehlbeeren sind erfindungsgemäß die Beeren der Pflanzen der Gattung *Sorbus* zu verstehen. Insbesondere bezeichnet der Begriff "Mehlbeeren" die Beeren der folgenden Bäume: Vogelbeere (Eberesche; *Sorbus aucuparia*), Speierling (*Sorbus domestica*), Elsbeere (*Sorbus torminalis*).

Wacholder bezeichnet im Sinne der Erfindung alle Arten der Gattung *Juniperus,* insbesondere den Gemeinen Wacholder (*Juniperus communis L.)* und den Sadebaum (*Juniperus sabina*).

Sanddorn bezeichnet erfindungsgemäß Pflanzen der Gattung *Hippophae,* insbesondere *Hippophae rhamnoides.*

"Aromastoffe" sind die für den charakteristischen Geschmack und Geruch der jeweiligen Beeren **B** verantwortlichen chemischen Verbindungen. Diese Aromen werden je nach Pflanzenart durch wenige Dutzend bis mehreren hundert chemische Verbindungen gebildet. Diese chemischen Verbindungen sind insbesondere Kohlenwasserstoffe (Terpene und Sesquiterpene) und oxygenierte Verbindungen (Alkohole, Aldehyde, Ketone, Säuren, Phenole, Laktone, Acetale, Ether und Ester).

Im Falle des Wacholders sind "Aromastoffe" insbesonders α-Pinen, β-Pinen, Borneol, Geraniol, Sabinen (CAS-Nummern: 3387-41-5; 2009-00-9; 10408-16-9), 3-Caren (CAS-Nummern: 13466-78-9; 498-15-7). Diese Aromastoffe finden sich auch teilweise in der Hagebutte, in Mehlbeeren und in Sanddornbeeren.

In dem ersten Schritt a. des erfindungsgemäßen Verfahrens werden die gegebenenfalls zerkleinerten Beeren mit überkritischem CO₂ ohne Zusatz eines Kosolvens extrahiert. Die Extraktion mit überkritischem CO₂ ist dabei wesentlich - mit unterkritischem CO₂ wäre eine ausreichende Extraktion von vorneherein nicht zu erreichen. Dabei werden die Beeren **B** entweder unzerkleinert oder zerkleinert, bevorzugt jedoch zerkleinert, eingesetzt. Werden die Beeren **B** zerkleinert eingesetzt, werden sie insbesondere in einem dem Schritt a. des erfindungsgemäßen Verfahrens vorgelagerten Schritt zerkleinert. Zur Zerkleinerung können dem Fachmann bekannte Verfahren genutzt werden, zum Beispiel Mahlen in der Mühle oder Quetschen im Walzenstuhl.

Im ersten Schritt a. des erfindungsgemäßen Verfahrens werden die zerkleinerten oder unzerkleinerten Beeren **B** mit überkritischem CO₂ ohne Zusatz eines Kosolvens extrahiert. Dazu weist das überkritische CO₂ insbesondere eine Temperatur im Bereich von 31 °C bis < 100 °C und einen Druck im Bereich von 74 bar bis 400 bar. Deshalb wird der Schritt a. insbesondere bei einer Temperatur im Bereich 31 °C bis < 100 °C und einen Druck im Bereich von 74 bar bis 400 bar durchgeführt, bevorzugt bei einer Temperatur im Bereich 40 °C bis 90 °C und einen Druck im Bereich von 100 bar bis 350 bar durchgeführt, bevorzugter bei einer Temperatur im Bereich 40 °C bis 70 °C und einen Druck im Bereich von 200 bar bis 300 bar durchgeführt, noch bevorzugter bei einer Temperatur im Bereich 40 °C bis 50 °C und einen Druck im Bereich von 250 bar bis 260 bar, ganz besonders bevorzugt bei 260 bar und 40 °C durchgeführt.

Die Extraktion der unzerkleinerten oder zerkleinerten Beeren **B** kann mit dem Fachmann bekannten Methoden durchgeführt werden. Beispielsweise beschreiben die EP 0 154 258 B1, die EP 0 200 150, die DE 34 15 844 A1 und die EP 1 815 899 A1 entsprechende Vorrichtungen zur Extraktion unter Verwendung überkritischen CO₂.

Die Menge an im Schritt a. des erfindungsgemäßen Verfahrens eingesetzten überkritischen CO₂ ist dabei nicht besonders beschränkt. Das Verhältnis des Gesamtgewichts an im Schritt a. des erfindungsgemäßen Verfahrens ohne Zusatz eines Kosolvens eingesetzten überkritischen CO₂ bezogen auf das Gesamtgewicht der im Schritt a. eingesetzten unzerkleinerten oder zerkleinerten Beeren **B** liegt insbesondere im Bereich 1 : 1 bis 100 : 1, bevorzugt im Bereich 10 : 1 bis 60 : 1, bevorzugter im Bereich 20: 1 bis 45 : 1.

Erfindungswesentlich ist, dass in dem ersten Schritt a. des erfindungsgemäßen Verfahrens überkritisches CO₂ ohne Zusatz eines Kosolvens eingesetzt wird. "Ohne Zusatz eines Kosolvens" bedeutet insbesondere, dass das Gesamtgewicht aller Kosolventien **K** (wobei **K** insbesondere ausgewählt aus der Gruppe bestehend aus Alkan, chloriertem Alkan, Alkohol, Alkancarbonsäureester, aliphatisches Keton, aliphatischer Ether, Wasser ist) bezogen auf das Gewicht des in Schritt a. eingesetzten überkritischen CO₂ geringer als 0.4 Gew.-%, bevorzugt geringer als 0.1 Gew.-%, noch bevorzugter geringer als 0.01 Gew.-% ist. Bevorzugter bedeutet "ohne Zusatz eines Kosolvens", dass das Gesamtgewicht aller von CO₂ verschiedener Stoffe bezogen auf das Gewicht des in Schritt a. eingesetzten überkritischen CO₂ geringer als 0.4 Gew.-%, bevorzugt geringer als 0.1 Gew.-%, noch bevorzugter geringer als 0.01 Gew.-% ist.

Nach Abschluss des ersten Schrittes a. des erfindungsgemäßen Verfahrens wird dann ein erster Extrakt umfassend überkritisches CO₂ und Aromastoffe erhalten. Daneben umfasst der erste Extrakt insbesondere Schleimstoffe und Zucker, die aus den Beeren **B** mitextrahiert wurden. Daneben wird auch ein fester Extraktionsrückstand **B_{Ex}** erhalten, also die extrahierten Beeren **B.** Dieser kann der Verbrennung oder Kompostierung, insbesondere der Kompostierung, zugeführt werden.

Es versteht sich von selbst, dass das erfindungsgemäße Verfahren auch den Fall umfasst, in dem während der Extraktion im Schritt a. des erfindungsgemäßen Verfahrens neben den Aromastoffen natürliche Feuchtigkeit aus den Beeren **B** teilweise in das überkritische CO₂ übergeht und der nach Durchführung des Schrittes a. erhaltene erste Extrakt zum Beispiel aus den Beeren **B** mitextrahiertes Wasser aufweist. Der erste Extrakt kann demnach auch Wasser aufweisen, bevorzugt weniger als 1 Gew.-% bezogen auf das Gesamtgewicht an CO₂ im ersten Extrakt.

Der Einsatz von CO₂ ohne Zusatz eines Kosolvens in Schritt a. macht das erfindungsgemäße Verfahren wirtschaftlicher und ergibt daneben einen ökologisch unbedenklichen Extraktionsrückstand **B_{EX}.**

In einem zweiten Schritt b. wird dann eine zumindest teilweise Abscheidung von CO₂ aus dem ersten Extrakt durchgeführt, wodurch ein zweiter Extrakt, welcher die Aromastoffe und eine gegenüber dem ersten Extrakt verringerte Menge an CO₂ umfasst, erhalten wird. "Zumindest teilweise Abscheidung von CO₂ aus dem ersten Extrakt" bedeutet insbesondere, dass mindestens 10 Gew.-%, insbesondere mindestens 25 Gew.-%, bevorzugt mindestens 50 Gew.-%, bevorzugter mindestens 75 Gew.-%, noch bevorzugter mindestens 85 Gew.-%, sogar noch bevorzugter mindestens 95 Gew.-%, am bevorzugtesten mindestens 98 Gew.-% des im ersten Extrakt umfassten CO₂ aus diesem entfernt werden. Vorzugsweise wird in Schritt b. des erfindungsgemäßen Verfahrens das CO₂ vollständig aus dem ersten Extrakt entfernt und es wird ein zweiter Extrakt erhalten, der neben den Aromastoffen insbesondere kein CO₂ mehr umfasst.

Die Abscheidung des CO₂ kann dabei nach dem Fachmann geläufigen Verfahren geschehen. Insbesondere kann dies einfach dadurch geschehen, dass man den ersten Extrakt Temperatur-Druckbedingungen aussetzt, bei welchem das vom ersten Extrakt umfasste CO₂ in den gasförmigen Zustand übergeht, die die Aromastoffe umfassende Phase aber im flüssigen Zustand vorliegt. Durch Veränderung der Druck- und/oder Temperaturbedingungen verändern sich dann auch die Lösungseigenschaften des Gases, und die vorher gelösten Stoffe werden dann zum Beispiel in einem Abscheidebehälter abgeschieden.

Wie in der EP 0 154 258 B1 ist dies durch die folgenden drei, auch im Rahmen der vorliegenden Erfindung einsetzbaren, Möglichkeiten zu erreichen:
A) in Schritt b. wird ein Druck unterhalb des kritischen Druckes des CO₂ (p_{Krit} = 73.75 bar) und eine Temperatur oberhalb der kritischen Temperatur des CO₂ (T_{Krit} = 30.980 °C) eingestellt;
B) in Schritt b. wird ein Druck unterhalb des kritischen Druckes des CO₂ (p_{Krit} = 73.75 bar) und eine Temperatur unterhalb der kritischen Temperatur des CO₂ (T_{Krit} = 30.980 °C) eingestellt;
C) in Schritt b. bleibt der Druck konstant wie in Schritt a., aber die Temperatur wird erhöht.

Die Möglichkeiten A) und B) sind im Schritt b) des erfindungsgemäßen Verfahrens dabei bevorzugt.

Dies ist insbesondere dadurch zu erreichen, dass der erste Extrakt einer Temperatur von > 0°C bis < 100 °C und einem Druck von 1 bar bis 72.9 bar ausgesetzt wird. Bevorzugt stellt man eine Temperatur von 10 °C bis 80 °C und einen Druck von 10 bar bis 65 bar ein, noch bevorzugter stellt man eine Temperatur von 30 °C bis 60 °C und einen Druck von 40 bar bis 60 bar ein, noch bevorzugter einen Temperatur von 30 °C und einen Druck von 45 bar bis 50 bar.

Das abgeschiedene CO₂ kann danach wieder in den flüssigen Zustand überführt und in einen Vorratsbehälter überführt und danach im überkritischen Zustand in den Extraktionskreislauf zurückgeführt werden (beispielsweise über eine Pumpe).

Der dann übrig gebliebene zweite Extrakt kann dann aus dem unter Druck stehenden Abscheider entlassen werden.

Nach Durchführung des Schrittes b wird ein zweiter Extrakt erhalten, der die Aromastoffe und eine gegenüber dem ersten Extrakt verringerte Menge an CO₂, bevorzugt kein CO₂ mehr, umfasst. Wie schon der erste Extrakt umfasst insbesondere auch der zweite Extrakt noch Schleimstoffe und Zucker, die aus den Beeren **B** mitextrahiert wurden.

Der nach Durchführung des Schrittes b. erhaltene zweite Extrakt weist die Aromastoffe und eine gegenüber dem ersten Extrakt verringerte Menge an CO₂, vorzugsweise die Aromastoffe und kein CO₂ auf. "Kein CO₂" bedeutet, dass der Gehalt an CO₂ im in Schritt b. erhaltenen zweiten Extrakt < 1 Gew.-%, insbesondere < 0.1 Gew.-%, bevorzugt < 0.01 Gew.-%, bevorzugter < 0.001 Gew.-%, noch bevorzugter < 0.0001 Gew.-% ist, wobei Gew.-% in dem Fall das Gesamtgewicht des im zweiten Extrakt enthaltenen CO₂ bezogen auf das Gesamtgewicht des zweiten Extrakts bedeutet.

Im Schritt c. des erfindungsgemäßen Verfahrens wird der in Schritt b. erhaltene zweite Extrakt dann mit einem Extraktionsmittel ausgewählt aus der Gruppe bestehend aus Alkan, chloriertem Alkan, Alkohol, Alkancarbonsäureester, aliphatisches Keton oder aliphatischer Ether extrahiert, wodurch der Extrakt **E**_{B} erhalten wird.

Insbesondere wird der in Schritt b. erhaltene zweite Extrakt im Schritt c. des erfindungsgemäßen Verfahrens dann mit einem Extraktionsmittel ausgewählt aus der Gruppe bestehend aus Pentan, Hexan, Methylenchlorid, Dichlorethylen, Trichlorethylen, Methanol, Ethanol, Propanol, Butanol, Essigsäuremethylester, Essigsäureethylester, Essigsäureisopropylester, Aceton, Acetylaceton, Dimethylether, Diethylether, Diisopropylether extrahiert.

Bevorzugt wird der in Schritt b. erhaltene Extrakt im Schritt c. des erfindungsgemäßen Verfahrens dann mit einem Extraktionsmittel ausgewählt aus der Gruppe bestehend aus Hexan, Ethanol, Propanol, Essigsäuremethylester extrahiert.

Bevorzugter wird der in Schritt b. erhaltene Extrakt im Schritt c. des erfindungsgemäßen Verfahrens dann mit einem Extraktionsmittel ausgewählt aus der Gruppe bestehend aus Hexan, Ethanol extrahiert.

Noch bevorzugter wird der in Schritt b. erhaltene Extrakt im Schritt c. des erfindungsgemäßen Verfahrens dann mit Hexan extrahiert.

Die Aromastoffe gehen dann im Schritt c. des erfindungsgemäßen Verfahrens in das Extraktionsmittel, welches in Schritt c. eingesetzt wird, über, während Schleimstoffe, Zucker ausfallen bzw. im Rückstand zurückbleiben. Die vom Extraktionsmittel gebildete Phase kann dann vom Rückstand abgetrennt werden. Das organische Lösungsmittel kann nach Abtrennung durch dem Fachmann geläufige Methoden (beispielsweise Destillation) entfernt werden, wodurch der gewünschte Extrakt **E**_{B} erhalten wird.

Das Verhältnis von in Schritt c. eingesetzten Extraktionsmittel ist dabei nicht besonders beschränkt und beträgt bevorzugt 20 : 1 bis 1 : 20 (Verhältnis Gesamtgewicht zweiter Extrakt zu Extraktionsmittel), bevorzugter 1: 1 bis 1: 20, noch bevorzugter 1 : 2 bis 1 : 10, noch bevorzugter 1 : 3 bis 1 : 8, noch bevorzugter 1 : 4 bis 1 : 6.

Es wurde überraschend festgestellt, dass durch den Schritt c. des erfindungsgemäßen Verfahrens, also Extraktion des in Schritt b. erhaltenen Extraktes, ein dritter Extrakt **E_{B}** erhalten wird, der die Aromastoffe von Beeren **B** ausgewählt aus Wacholderbeeren, Sanddornbeeren, Hagebutten, Mehlbeeren umfasst, aber einen gegenüber den in der EP 0 154 258 B1 erhaltenen Extrakt einen geringeren Zuckergehalt aufweist.

Zusätzlich wurde festgestellt, dass die nötige Entfernung der Schleimstoffe ebenfalls im Schritt c. erreicht werden kann, wobei durch die Kombination aus Schritten a. und c. des erfindungsgemäßen Verfahrens die zur Erreichung einer ebenso großen Entschleimung wie in der EP 0 154 258 B1 eine sehr viel geringere Menge an organischem Extraktionsmittel nötig ist. Dies war ebenfalls völlig überraschend.

Schließlich ist der Rückstand **B_{Ex}** der Beeren **B,** welcher in Schritt a. zurückbleibt, nicht mit organischem Lösungsmittel belastet (da dieser Schritt ja ohne Zusatz von Kosolvens durchgeführt wurde). Dadurch kann der Rückstand an extrahierten Beeren der Kompostierung zugeführt werden.

Schließlich wurde festgestellt, dass der mit dem erfindungsgemäßen Verfahren erhaltene Extrakt **E_{B}** gegenüber dem in der EP 0 154 258 B1 erhaltenen von besserer Qualität, da viel weniger gefärbt und weniger mit organischem Lösungsmittel wie zum Beispiel Hexan belastet ist.

Das erfindungsgemäße Verfahren kann kontinuierlich oder im Batchbetrieb, bevorzugt jedoch kontinuierlich, durchgeführt werden.

Die folgenden Beispiele sollen die Erfindung erläutern, ohne diese auf diese Beispiele zu beschränken.

### Beispiele

### Erfinderisches Beispiel E1

Im Walzenstuhl gequetschte Wacholderbeeren (70 kg) wurden in den Autoklaven gefüllt und dieser verschlossen. Mittels einer Hochdruckpumpe wurde der Autoklav nun auf 260 bar aufgedrückt und die Extraktionstemperatur auf 40 °C eingestellt. Es wird von unten nach oben im Kreislaufbetrieb für 2 Stunden mit überkritischem CO₂ extrahiert. Für 1 kg Beeren wurden dabei 45 kg CO₂ benötigt (S/F 45). Während des Kreislauffahrens wird der Extrakt im Abscheider aufgefangen. Nach dem Ende der Extraktion wird unten am Abscheider zuerst das Wasser abgelassen, anschließend der Wacholderextrakt.

Der erhaltene Extrakt (8.6 kg) enthält unter anderem Fette, Zucker, Schleimstoffe und Aromastoffe und ist trübe. Der Rückstand der extrahierten Wacholderbeeren kann der Kompostierung zugeführt werden.

Zur Herstellung eines klaren, niedrigviskosen und hellgrünen Extrakts wird der erhaltene Extrakt mit der 4-fachen Masse an Hexan intensiv gerührt. Nach 24 - stündigen Stehenlassen wurde der klare Überstand in den Dünnschichtverdampfer eingespeist. Hier erfolgte unter Temperatur und Vakuum die Abtrennung des Hexans vom Wacholderextrakt.

Der übrig gebliebene Bodensatz wurde mit der doppelten Menge Hexans nochmals intensiv gerührt und bis zur Bildung eines klaren Überstands stehen gelassen. Der Überstand wird abfiltriert und im Dünnschichtverdampfer vom Hexan befreit. Der Wacholderextrakt wird mit dem vorhergehenden Extrakt vereinigt. Der verbliebene Bodensatz wurde der Verbrennung zugeführt.

Man erhält aus 70 kg Ausgangsmaterial 7.3 kg eines klaren Extraktes aus Wacholderbeeren. Dieser Extrakt weist weniger als 1000 ppm Hexan auf (bestimmt durch gaschromatographische Messungen).

### Vergleichsbeispiel V1

Entsprechend dem Beispiel 1 a) der EP 0 154 258 B1 wurden im Walzenstuhl gequetschte Wacholderbeeren (60 kg) in den Autoklaven gefüllt und dieser verschlossen. Mittels einer Hochdruckpumpe wurde der Autoklav nun auf 260 bar aufgedrückt und die Extraktionstemperatur auf 40 °C eingestellt. Es wurde von unten nach oben im Kreislaufbetrieb mit überkritischem CO₂ extrahiert, welchem in einer Schleppmittelkonzentration von 3.4 % Hexan zugegeben wurde.

Es wird von unten nach oben im Kreislaufbetrieb für 2 Stunden mit überkritischem CO₂ enthaltend das Kosolvens Hexan extrahiert. Für 1 kg Beeren wurden dabei 45 kg CO₂ benötigt (S/F 45). Während des Kreislauffahrens wird der Extrakt im Abscheider aufgefangen. Nach dem Ende der Extraktion wird unten am Abscheider der Wacholderextrakt abgelassen.

Der Wacholderextrakt wurde sedimentiert und filtriert. Um das Hexan bis auf einen Gehalt von weniger als 1000 ppm (bestimmt durch gaschromatographische Messungen) zu entfernen, war es nötig, den Extrakt drei Tage bei 65 °C im Vakuum zu behandeln. Es wurden 5.4 kg Wacholderextrakt gewonnen. Der Rückstand der extrahierten Wacholderbeeren ist mit Hexan belastet und muss der Verbrennung zugeführt werden. Eine Kompostierung ist aus ökologischen Gründen nicht möglich.

Die folgende Tabelle gibt außerdem die für die Extraktion von 70 kg Wacholderbeeren benötigte Menge an Hexan wieder.

| Versuch | Hexan (g/ kg Wacholder) |
|---|---|
| **V1** | 110 |
| **E1** | 20 |

Der in **V1** erhaltene Extrakt ist süß, dunkel gefärbt und hochviskos. Der in **E1** erhaltene Extrakt ist deutlich klar, hellgrün, weniger süß und niedrigviskos.

Die Versuche können mit Ethanol statt Hexan wiederholt werden und führen dann zu analogen Ergebnissen.

Aus den vorstehenden Versuchen ist ersichtlich:
1. Mit dem erfinderischen Verfahren ist der Erhalt eines Extraktes, dessen Zuckergehalt weit unter dem der nach den Verfahren des Standes der Technik erhältlichen liegt und welcher zusätzlich reiner ist, möglich.
2. Mit dem erfindungsgemäßen Verfahren ist die Extraktion unter geringerem Einsatz an organischem Extraktionsmittel möglich.
3. Das Verfahren ist umweltfreundlicher, da der feste Rückstand der mit CO₂ extrahierten Wacholderbeeren nicht mit Hexan belastet ist.
4. Die Entfernung von Hexan aus dem Extrakt aus **E1** ist deutlich weniger aufwändig als die Entfernung von Hexan aus dem in **V1** gewonnenen Extrakt, da der gewünschte Gehalt an Hexan von weniger als 1000 ppm schon durch kontinuierliche Dünnschichtverdampfung in **E1** erreicht wird, während in **V1** dieser Gehalt an Hexan im Extrakt nur mittels eines zusätzlichen Verfahrensschrittes der Vakuumdestillation erhältlich ist.

## Patentansprüche

1. Verfahren zur Gewinnung eines Extraktes **E_{B}** umfassend Aromastoffe von Beeren **B** ausgewählt aus Wacholderbeeren, Sanddornbeeren, Hagebutten, Mehlbeeren, wobei man
a. die zerkleinerten oder unzerkleinerten Beeren **B** mit überkritischem CO₂ ohne Zusatz eines Kosolvens extrahiert, wodurch ein fester Extraktionsrückstand **B_{Ex}** sowie ein erster Extrakt umfassend überkritisches CO₂ und Aromastoffe erhalten werden;
b. mindestens teilweise CO₂ aus dem in Schritt a. erhaltenen ersten Extrakt entfernt, wodurch ein zweiter Extrakt, welcher die Aromastoffe und eine gegenüber dem ersten Extrakt verringerte Menge an CO₂ umfasst, erhalten wird;
c. den in Schritt b. erhaltenen zweiten Extrakt mit mindestens einem Extraktionsmittel ausgewählt aus der Gruppe bestehend aus Alkan, chloriertem Alkan, Alkohol, Alkancarbonsäureester, aliphatisches Keton, aliphatischer Ether extrahiert, wodurch der Extrakt **E_{B}** erhalten wird.

2. Verfahren nach Anspruch 1, wobei die Beeren **B** Wacholderbeeren sind.

3. Verfahren nach einem der Ansprüche 1 oder 2, wobei man in Schritt b. das CO₂ dadurch zumindest teilweise aus dem ersten Extrakt entfernt, dass der erste Extrakt einer Temperatur von > 0°C bis < 100 °C und einem Druck von 0.1 bar bis 72.9 bar ausgesetzt wird.

4. Verfahren nach einem der Ansprüche 1 bis 3, wobei das Extraktionsmittel in Schritt c. ausgewählt ist aus der Gruppe bestehend aus Hexan, Ethanol, Propanol, Methylacetat.

5. Verfahren nach Anspruch 4, wobei das Extraktionsmittel in Schritt c. Hexan ist.

6. Verfahren nach Anspruch 4, wobei das Extraktionsmittel in Schritt c. Ethanol ist.

7. Verfahren nach einem der Ansprüche 1 bis 6, wobei der Schritt a. bei einer Temperatur im Bereich 31 °C bis < 100 °C und einen Druck im Bereich von 74 bar bis 400 bar durchgeführt wird.

8. Verfahren nach einem der Ansprüche 1 bis 7, wobei die Beeren **B** in Schritt a. zerkleinert eingesetzt werden.

9. Verfahren nach einem der Ansprüche 1 bis 8, welches kontinuierlich durchgeführt wird.

## Claims

1. Process for obtaining an extract **E_{B}** comprising flavourings from berries **B** selected from among juniper berries, seabuckthorn berries, rosehips, Sorbus, wherein
a. the comminuted or non-comminuted berries **B** are extracted with supercritical CO₂ without addition of a cosolvent, giving a solid extraction residue **B_{Ex}** and a first extract comprising supercritical CO₂ and flavourings;
b. at least some of the CO₂ is removed from the first extract obtained in step a., which gives a second extract which comprises the flavourings and an amount of CO₂ which is reduced in comparison with the first extract;
c. the second extract, which is obtained in step b., is extracted with at least one extractant selected from the group consisting of alkane, chlorinated alkane, alcohol, alkane carboxylic ester, aliphatic ketone, aliphatic ether, which gives the extract **E_{B}.**

2. Process according to Claim 1, wherein the berries **B** are juniper berries.

3. Process according to one of Claims 1 or 2, wherein, in step b., at least some of the CO₂ is removed from the first extract by exposing the first extract to a temperature of from > 0°C to < 100°C and to a pressure of from 0.1 bar to 72.9 bar.

4. Process according to one of Claims 1 to 3, wherein the extractant in step c. is selected from the group consisting of hexane, ethanol, propanol, methyl acetate.

5. Process according to step 4, wherein the extractant in step c. is hexane.

6. Process according to step 4, wherein the extractant in step c. is ethanol.

7. Process according to one of Claims 1 to 6, wherein step a. is carried out at a temperature in the range of from 31°C to < 100°C and a pressure in the range of from 74 bar to 400 bar.

8. Process according to one of Claims 1 to 7, wherein the berries **B** in step a. are employed in comminuted form.

9. Process according to one of Claims 1 to 8, which is carried out continuously.

## Revendications

1. Procédé d'obtention d'un extrait E_{B} comprenant des arômes de baies B choisies parmi les baies de genévrier, les baies d'argousier, les fruits de l'églantier, les baies de sorbier, dans lequel
a. les baies B broyées ou non broyées sont extraites avec du CO₂ supercritique sans ajout d'un co-solvant, un résidu d'extraction solide B_{Ex} et un premier extrait comprenant du CO₂ supercritique et des arômes étant obtenus ;
b. le CO₂ est éliminé au moins partiellement du premier extrait obtenu à l'étape a., un deuxième extrait, qui comprend les arômes et une quantité de CO₂ réduite par rapport au premier extrait, étant ainsi obtenu ;
c. le deuxième extrait obtenu à l'étape b. est extrait avec au moins un agent d'extraction choisi dans le groupe constitué par un alcane, un alcane chloré, un alcool, un ester d'acide alcane-carboxylique, une cétone aliphatique, un éther aliphatique, l'extrait E_{B} étant ainsi obtenu.

2. Procédé selon la revendication 1, dans lequel les baies B sont des baies de genévrier.

3. Procédé selon l'une quelconque des revendications 1 ou 2, dans lequel, à l'étape b., le CO₂ est éliminé au moins partiellement du premier extrait par exposition du premier extrait à une température de > 0 °C à < 100 °C et à une pression de 0,1 bar à 72,9 bar.

4. Procédé selon l'une quelconque des revendications 1 à 3, dans lequel l'agent d'extraction à l'étape c. est choisi dans le groupe constitué par l'hexane, l'éthanol, le propanol, l'acétate de méthyle.

5. Procédé selon la revendication 4, dans lequel l'agent d'extraction à l'étape c. est l'hexane.

6. Procédé selon la revendication 4, dans lequel l'agent d'extraction à l'étape c. est l'éthanol.

7. Procédé selon l'une quelconque des revendications 1 à 6, dans lequel l'étape a. est réalisée à une température dans la plage allant de 31 °C à < 100 °C et à une pression dans la plage allant de 74 bar à 400 bar.

8. Procédé selon l'une quelconque des revendications 1 à 7, dans lequel les baies B sont utilisées broyées à l'étape a.

9. Procédé selon l'une quelconque des revendications 1 à 8, qui est réalisé en continu.
